# EUROPEAN PATENT SPECIFICATION

(11) **EP 2 542 080 B1**
(45) Date of publication and mention of the grant of the patent: **31.08.2016**
(21) Application number: 11751305.1
(22) Date of filing: 02.03.2011
(51) Int. Cl.: A61K 31/4704, A61P 37/06, A61P 19/02, A61K 45/06

(54) **TREATMENT OF LUPUS ARTHRITIS USING LAQUINIMOD**
BEHANDLUNG VON LUPUS ARTHRITIS MIT LAQUINIMOD
TRAITEMENT DE L'ARTHRITE DE LUPUS À L'AIDE DU LAQUINIMOD

(30) Priority: 03.03.2010 US 339355 P
(43) Date of publication of application: 09.01.2013
(73) Proprietor: Teva Pharmaceutical Industries Ltd., 49131 Petach-Tikva (IL)
(72) Inventor: HAVIV, Asi, 76802 Gan Shlomo (IL); TARCIC, Nora, Modiin (IL)
(74) Representative: D Young & Co LLP
(86) International application number: PCT/US2011/026891
(87) International publication number: WO 2011/109536

(56) References cited:
- WO-A2-2007/139887
- US-A- 6 077 851
- US-A- 6 077 851
- US-A1- 2007 086 979
- US-A1- 2007 293 537
- US-A1- 2009 221 575
- GROSSMAN ET AL: "Lupus arthritis", BAILLIERE'S BEST PRACTICE AND RESEARCH. CLINICAL REUMATOLOGY, BAILLIERE TINDALL, LONDON, GB, vol. 23, no. 4, 1 August 2009 (2009-08-01) , pages 495-506, XP026281064, ISSN: 1521-6942, DOI: 10.1016/J.BERH.2009.04.003 [retrieved on 2009-07-08]
- JANA PREININGEROVA: "Oral laquinimod therapy in relapsing multiple sclerosis", EXPERT OPINION ON INVESTIGATIONAL DRUGS, INFORMA HEALTHCARE, UK, vol. 18, no. 7, 1 July 2009 (2009-07-01), pages 985-989, XP008157941, ISSN: 1354-3784, DOI: 10.1517/13543780903044944

## Description

### Background

Lupus arthritis, characterized by inflammation and pain of body joints, is a complication which occurs in a subpopulation of patients with Systemic Lupus Erythematosus (SLE) and is the most common cause of joint pain in lupus sufferers.

SLE is a debilitating autoimmune disease of great clinical diversity and can manifest itself in different ways and lead to a number of complications, e.g., arthritis, arthralgia, and myalgia, depending on the patient and the parts of the body affected. The precise etiology of SLE has not yet been determined, but hormonal, genetic, viral and environmental factors may precipitate the disease. SLE prevalence varies across ethnicities and geographic regions with an occurrence rate of 15 to 50 cases per 100,000 persons (Bevra, 2001). SLE is most common in women of childbearing age (15-44) with a female-to-male ratio varying from 4.3 to 13.6 (Petri, 2002). Virtually all body systems may be involved, including the musculoskeletal, mucocutaneous, cardiovascular, neurological, respiratory, renal, ophthalmic hematological and gastrointestinal systems.

Due to the great clinical diversity and idiopathic nature of SLE, management of idiopathic SLE depends on its specific manifestations and severity. (The Merck Manual, 1999) Therefore, medications suggested to treat SLE generally are not necessarily effective for the treatment of all manifestations of and complications resulting from SLE, e.g., lupus arthritis.

Joints, muscles and their supporting structures are the most commonly involved system in SLE, affecting 53-95% of patients (Wallace, 2007). More than 90% of people with SLE will experience joint and/or muscle pain at some time during the course of their illness, while the prevalence of arthritis among SLE patients is estimated as above 50% (Wallace, 2007).

The American College of Rheumatology established eleven criteria in 1982, which were revised in 1997, as a classificatory instrument to operationalise the definition of SLE in clinical trials. American College of Rheumatology Revised Classification Criteria for Systemic Lupus Erythematosus, defines lupus arthritis is "nonerosive arthritis of two or more peripheral joints, with tenderness, swelling, or effusion."

Lupus arthritis causes pain, stiffness, swelling, tenderness, and warmth in the joints in a waxing and waning pattern. The joints most often affected are the ones farthest from the middle of the body, such as fingers, wrists, elbows, knees, ankles, and toes. General stiffness when upon waking up in the morning, which gradually improves as the day goes on, is a key feature of lupus arthritis. However, joint pain may occur later in the day.

In lupus arthritis, several joints are usually involved, and the inflammation will affect similar joints on both sides of the body. All major and minor joints may be affected. Compared to rheumatoid arthritis, however, lupus arthritis is less disabling and less likely to cause destruction of the joints. Fewer than 10 percent of people with lupus arthritis will develop deformities of their hands and feet associated with weakening of cartilage and bone.

Although joints, muscles and their supporting structures are the most commonly involved system in SLE, very few clinical trials, performed to date, primarily assessed this organ system response to treatment.

There is no definitive treatment or cure for lupus arthritis. The principal goals of therapy are to relieve symptoms and improve function. According to the Lupus Foundation of America, current treatment for lupus arthritis has five basic goals: reduce inflammation, suppress immune system, prevent and treat flare ups of the condition, control symptoms and limit any damage to the body and organs.

Nonsteroidal anti-inflammatories (NSAIDs), corticosteroids, antimalarials and a variety of immunosuppressive medications are the standard of care for patients with lupus arthritis. (Grossman, 2009) To reduce joint stiffness and pain, low impact exercise has been recommended as well. Other than treating lupus arthritis all accompanied SLE signs, symptoms and complications should be treated.

While many patients fail to respond or respond only partially to the standard of care medications listed above, the long-term use of high doses of corticosteroids and immunosuppressive therapies may have profound side effects. Infectious complications coincident with active SLE and its treatment with immunosuppressive medications are the most common cause of death in patients with SLE.

There is, therefore, a definite need for alternative therapies with better risk-benefit profiles for the treatment of lupus arthritis.

### Summary of the Invention

This invention also provides laquinimod or pharmaceutically acceptable salt thereof for use in treating a subject afflicted with active lupus arthritis.

This invention further provides a pharmaceutical composition comprising an amount of laquinimod or pharmaceutically acceptable salt thereof for use in treating a subject afflicted with active lupus arthritis.

### Detailed Description of the invention

This invention provides laquinimod or pharmaceutically acceptable salt thereof or a pharmaceutical composition comprising it for use in a method of treating a subject afflicted with active lupus arthritis said treatment comprising periodically administering to the subject an amount of laquinimod or pharmaceutically acceptable salt thereof effective to treat the subject.

In one embodiment, the amount of laquinimod or pharmaceutically acceptable salt thereof is effective to reduce a clinical sign or symptom of active lupus arthritis in the subject. In another embodiment, the pharmaceutically acceptable salt of laquinimod is laquinimod sodium.

In one embodiment, the periodic administration of laquinimod or pharmaceutically acceptable salt thereof is effected orally. In another embodiment, the amount of laquinimod administered is 0.5-1.0 mg/day. In another embodiment, the amount of laquinimod administered is 0.5 mg/day. In yet another embodiment, the amount of laquinimod administered is 1.0 mg/day.

In one embodiment the treatment further comprises administration of corticosteroids, immunosuppressives, anti-malarial drugs, non steroid anti-inflammatory drugs and/or COX2 inhibitors.

In one embodiment, the periodic administration continues for at least 12 weeks.

In one embodiment, the laquinimod or pharmaceutically acceptable salt thereof is administered as monotherapy for lupus arthritis. In another embodiment, the laquinimod or pharmaceutically acceptable salt thereof is administered as adjunct therapy with another lupus arthritis treatment.

In one embodiment, the periodic administration of laquinimod or pharmaceutically acceptable salt thereof reduces the subject's swollen joint count. In another embodiment, the periodic administration of laquinimod or pharmaceutically acceptable salt thereof reduces the subject's tender joint count. In yet another embodiment, the periodic administration of laquinimod or pharmaceutically acceptable salt thereof improves the subject's BILAG MSK response.

In one embodiment, the periodic administration of laquinimod or pharmaceutically acceptable salt thereof improves the subject's BILAG score.

In one embodiment, the periodic administration of laquinimod or pharmaceutically acceptable salt thereof lowers the C-Reactive Protein level, serum cytokine level, serum chemokine level and/or anti-dsDNA level of the subject. In another embodiment, the periodic administration of laquinimod or pharmaceutically acceptable salt thereof reduces the subject's Disease Activity Score using 28 joint counts (DAS28), 66 swollen/68 tender joint count (JC66/68) and/or reduces the subject's physician global assessment (PGA) score.

In one embodiment, the subject is human.

This invention also provides laquinimod or pharmaceutically acceptable salt thereof or a pharmaceutical composition comprising it for use in a method of treatino Active lupus arthritis in a subject afflicted therewith said treatment comprising periodically administering to the subject an amount of laquinimod or pharmaceutically acceptable salt thereof effective to treat the active lupus arthritis in the subject.

This invention also provides laquinimod or pharmaceutically acceptable salt thereof for use in treating a subject afflicted with active lupus arthritis.

This invention further provides a pharmaceutical composition comprising an amount of laquinimod or pharmaceutically acceptable salt thereof for use in treating a subject afflicted with lupus arthritis.

For the foregoing embodiments, each embodiment disclosed herein is contemplated as being applicable to each of the other disclosed embodiment.

It is understood that where a parameter range is provided, all integers within that range, and tenths thereof, are also provided by the invention. For example, "0.5-1 mg/day" includes 0.5 mg/day, 0.6 mg/day etc. up to 1.0 mg/day.

Disclosed is a method of treating a subject afflicted with lupus, specifically, lupus arthritis, using laquinimod. Laquinimod is a novel synthetic compound with high oral bioavailability which has been suggested as an oral formulation for the treatment of Multiple Sclerosis (MS), (Polman, 2005; Sandberg-Wollheim, 2005). Laquinimod and its sodium salt form are described, for example, in U.S. Patent No. 6,077,851. The effects of laquinimod on lupus arthritis have not been reported. As described herein, administration of laquinimod is effective to treat the subject afflicted with lupus arthritis.

Administration of laquinimod is advantageous over existing treatment for lupus arthritis because laquinimod can be administered orally and is not an immunosuppressant. In addition, laquinimod has a unique mechanism of action which contributes to potential additive effect when used in combination with standard of care.

### Terms

As used herein, and unless stated otherwise, each of the following terms shall have the definition set forth below.

As used herein, an "amount" or "dose" of laquinimod as measured in milligrams refers to the milligrams of laquinimod acid present in a preparation, regardless of the form of the preparation. Therefore, a "dose of 0.5 mg laquinimod" means the amount of laquinimod acid in a preparation is 0.5 mg, regardless of the form of the preparation. Similarly, a "dose of 1 mg laquinimod" means the amount of laquinimod acid in a preparation is 1 mg, regardless of the form of the preparation. Thus, when in the form of a salt, e.g. a laquinimod sodium salt, the weight of the salt form necessary to provide a dose of 0.5 mg laquinimod would be greater than 0.5 mg due to the presence of the additional salt ion.

As used herein, "laquinimod" means laquinimod acid or a pharmaceutically acceptable salt thereof.

As used herein, "a subject afflicted with active lupus arthritis" means a subject who was been affirmatively diagnosed to have active lupus arthritis.

As used herein, "joint tenderness" is defined as the presence of tenderness and/or pain in a joint at rest with pressure or on passive movement of the joint/joint manipulation.

As used herein, "joint swelling" is soft tissue swelling that is detectable along the joint margins.

As used herein, "effective" when referring to an amount of laquinimod refers to the quantity of laquinimod that is sufficient to yield a desired therapeutic response without undue adverse side effects (such as toxicity, irritation, or allergic response) commensurate with a reasonable benefit/risk ratio when used in the manner of this invention.

As used herein, "treating" encompasses, e.g., inducing inhibition, regression, or stasis of a disorder, or lessening, suppressing, inhibiting, reducing the severity of, eliminating, or ameliorating a symptom of the disorder.

As used herein, "inhibition" of disease progression or disease complication in a subject means preventing or reducing the disease progression and/or disease complication in the subject.

As used herein, a "symptom" associated with lupus arthritis includes any clinical or laboratory manifestation associated with lupus arthritis and is not limited to what the subject can feel or observe.

As used herein, "The British Isles Lupus Assessment Group Index" or "BILAG" index is a validated comprehensive computerized index for measuring clinical disease activity in systemic lupus erythematosus (SLE), which was developed according to the principle of the physician's invention to treat'.

A BILAG assessment consists of 97 variables, some based on the patient's history, some on examination findings and others on laboratory/imaging results. The questions are grouped under nine systems: Constitutional, Mucocutaneous, Neuropsychiatric, Musculoskeletal, Cardiorespiratory, Gastrointestinal, Ophthalmic, Renal and Hematological.

The index attempts to capture only SLE related disease activity in the previous 4 weeks prior to each assessment. Each of the clinical variables may be recorded as:
0. **Absent.**
1. **Improved.** Sufficient for considering reduction in therapy **and** [improvement present on assessment and for at least 2 weeks **or** completely resolved within the entire last week].
2. **Same.** No improvement and no deterioration within the last 4 weeks compared to the previous 4 weeks or improvement does not meet improvement criteria.
3. **Worse.** Deteriorated during the last 4 weeks compared to the previous 4 weeks.
4. **New.** New or recurrent episode during the last 4 weeks (compared to the previous 4 weeks), which is not improving.

Based upon the scoring to each of these variables, a pre-defined algorithm, specific for each system, provides a disease activity score ranging from A to E for each system:
Grade 'A' = severe disease activity requiring treatment with high dose steroids (>20mg/day oral prednisolone or equivalent or IV pulse >500 mg MP), systemic immunomodulators or high dose anticoagulation
Grade 'B' = moderate disease activity requiring treatment with low dose oral steroids (<20mg/day prednisolone or equivalent), IM or IA steroids (equivalent to MP<500 mg), topical steroids or immunomodulators, antimalarials or symptomatic therapy (e.g. NSAIDS).
Grade 'C' = mild disease.
Grade 'D' = indicates previously affected but currently inactive.
Grade 'E' = this system has never been involved.

As used herein, SLE disease activity index "SLEDAI 2K" is a validated tool developed as a global assessment of disease activity in SLE patients. It represents the consensus of a group of experts in the field of lupus research. The SLEDAI 2K assesses 24 descriptors (sixteen clinical manifestations and eight laboratory measures) in 9 organ systems. Descriptors are given different weights, based on clinical importance, with dichotomic score (present/not present within the previous 30 days). A descriptor must be attributed to active SLE or otherwise should not be scores. The SLEDAI 2K is intended to evaluate current lupus activity and not chronic damage.

As used herein, "Evaluator/physician Global Assessment (EGA)" is a Visual Analogue Scale. It measures the disease activity based on the physician subjective assessment from none active to worse disease activity. EGA is performed at every visit (except for screening).

As used herein, "Patient Global Assessment (PGA)" is a Visual Analogue Scale. It measures the subject perception of his/hers overall health condition, from very well to very poor. As used herein, an "adverse event" or "AE" means any untoward medical occurrence in a clinical trial subject administered a medicinal product and which does not have a causal relationship with the treatment. An adverse event can therefore be any unfavorable and unintended sign including an abnormal laboratory finding, symptom, or diseases temporally associated with the use of an investigational medicinal product, whether or not considered related to the investigational medicinal product.

As used herein, "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

When referring to dosing, the designation "BID" indicates that the dose is administered twice daily. The designation "QD" indicates that the dose is administered once daily.

A number of experiments were conducted testing for the effects of laquinimod on lupus manifestations using murine models. (see Examples 1.1-1.4) However, the effects of laquinimod on lupus arthritis in humans have not been reported. Therefore, based on the encouraging results of these experiments, a clinical trial is initiated (See Example 2).

The use of laquinimod for SLE had been previously suggested in, e.g., U.S. Patent No. 6,077,851. However, without empirical evidence, one cannot affirmatively establish that laquinimod will be effective for treating all complications arising from SLE based on this disclosure alone. The '851 patent does not disclose the use of laquinimod for the particular sub-population of SLE relevant to the instant invention. That is, the '851 patent does not disclose the use of laquinimod for lupus arthritis. On the other hand, the inventors have surprising found that laquinimod is particularly effective for the treatment of lupus arthritis.

A pharmaceutically acceptable salt of laquinimod as used in this application includes lithium, sodium, potassium, magnesium, calcium, manganese, copper, zinc, aluminum and iron. Salt formulations of laquinimod and the process for preparing the same are described, e.g., in U.S. Patent Application Publication No. 2005/0192315 and PCT International Application Publication No. WO 2005/074899, which are hereby incorporated by reference into this application.

A dosage unit may comprise a single compound or mixtures of compounds thereof. A dosage unit can be prepared for oral dosage forms, such as tablets, capsules, pills, powders, and granules.

Laquinimod can be administered in admixture with suitable pharmaceutical diluents, extenders, excipients, or carriers (collectively referred to herein as a pharmaceutically acceptable carrier) suitably selected with respect to the intended form of administration and as consistent with conventional pharmaceutical practices. The unit is preferably in a form suitable for oral administration. Laquinimod can be administered alone but is generally mixed with a pharmaceutically acceptable carrier, and co-administered in the form of a tablet or capsule, liposome, or as an agglomerated powder. Examples of suitable solid carriers include lactose, sucrose, gelatin and agar. Capsule or tablets can be easily formulated and can be made easy to swallow or chew; other solid forms include granules, and bulk powders. Tablets may contain suitable binders, lubricants, disintegrating agents, coloring agents, flavoring agents, flow-inducing agents, and melting agents. For instance, for oral administration in the dosage unit form of a tablet or capsule, the active drug component can be combined with an oral, non-toxic, pharmaceutically acceptable, inert carrier such as lactose, gelatin, agar, starch, sucrose, glucose, methyl cellulose, dicalcium phosphate, calcium sulfate, mannitol, sorbitol, microcrystalline cellulose and the like. Suitable binders include starch, gelatin, natural sugars such as glucose or beta-lactose, corn starch, natural and synthetic gums such as acacia, tragacanth, or sodium alginate, povidone, carboxymethylcellulose, polyethylene glycol, waxes, and the like. Lubricants used in these dosage forms include sodium oleate, sodium stearate, sodium benzoate, sodium acetate, sodium chloride, stearic acid, sodium stearyl fumarate, talc and the like. Disintegrators include, without limitation, starch, methyl cellulose, agar, bentonite, xanthan gum, croscarmellose sodium, sodium starch glycolate and the like.

Specific examples of the techniques, pharmaceutically acceptable carriers and excipients that may be used to formulate oral dosage forms of the present invention are described, e.g., in U.S. Patent Application Publication No. 2005/0192315, PCT International Application Publication Nos. WO 2005/074899, WO 2007/047863, and WO 2007/146248.

General techniques and compositions for making dosage forms useful in the present invention are described in the following references: 7 Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol. 40 (Gilbert S. Banker, Christopher T. Rhodes, Eds.). These references in their entireties are hereby incorporated by reference into this application.

This invention will be better understood by reference to the Experimental Details which follow, but those skilled in the art will readily appreciate that the specific experiments detailed are only illustrative of the invention as described more fully in the claims which follow thereafter.

### Experimental Details

### EXAMPLE 1: Assessment of The Effect of Laquinimod For SLE In Animal Models

Systemic Lupus Erythematosus (SLE) is a disorder of generalized autoimmunity characterized by defective T cell-mediated responses and the formation of a variety of antibodies reactive to self or altered self-antigens. SLE is mainly characterized by the presence of anti-DNA antibodies. Some of these auto-antibodies combine with the corresponding auto-antigens, forming immune complexes, either in the circulating blood or directly in tissues, resulting in severe damage. Glomerulonephritis induced by immune complexes is in fact the major cause of death in patients with SLE. (NZBxNZW)F1 are lupus-prone mice that develop an SLE-like disease spontaneously including anti-dsDNA antibodies (Abs), proteinuria and Immune Complex Deposits (ICD). The (NZBxNZW)F1 (NZB/W) murine model is the hallmark of spontaneous SLE.

In a number of studies, the effect of various doses of laquinimod in the (NZBxNZW)F1 model for SLE were assessed. The studies also included a negative control (water) and positive controls including cyclophosphamide (CTX) and methotrexate (MTX).

### Example 1.1 - Effect of Laquinimod, Cytoxan(CTX), and Methotrexate (MTX) on Lupus Manifestations Using the (NZBxNZW)F1 Mouse Model

This study investigated the effect of laquinimod, an immunomodulator of SLE in a murine model of SLE and compared the treatment effect to reference substances CTX and MTX. CTX is an alkylating agent that has become the standard of care for the disease management of most severe forms of lupus. MTX is an antimetabolite drug used in treatment of cancer and autoimmune diseases. It acts by inhibiting the metabolism of folic acid via the inhibition of dihydrofolate reductase and blocks DNA synthesis in rapidly proliferate cells. These actions include immunosuppression. Both CTX and MTX have shown efficacy in prior studies.

Laquinimod and reference compounds CTX and MTX were applied in therapeutic mode, starting the treatment at the time when the characteristic change of murine SLE model, proteinuria (PU) was present in > 80% of animals, and the observation and treatment period following this was 12 weeks. Laquinimod was applied p.o. daily, in a dose of 25 mg/kg. CTX was applied once weekly in a dose of 25 mg/ig i.p. MTX was applied 3 times a week p.o. at 35µg/mouse.

Also, body weight changes were recorded weekly and at the end of experiment both kidneys were preserved, one for possible conventional histology and one for immune complex detection *ICD) in glomeruli. Evaluation of ICD was performed by scoring and by image analysis.

80 animals were involved in the study. During the treatment period 4 animals died, 2 from the vehicle treated group and 2 from the MTX treated group.

The severity of disease followed by PU measurement showed gradual increase in the control (water treated, vehicle) group, but substantial difference between the treated and vehicle groups developed around the 8-12^{th} week of observation. At week 12 observation, laquinimod and CTX treatment significantly diminished the proteinuria (p <.01 and P < 0.05 by MW U test, respectively).

At the end of experiment ICD was evaluated by two methods, and the results from the two methods showed good correlation (correlation coefficient: 0.993). The immune complex deposition was significantly inhibited by laquinimod and CTX (p< 0.001 and p < 0.05, respectively) - the results correlate well with the PU data on the last week (correlation coefficient of group averages of ICD and PU: 0.8199).

Therefore, laquinimod and the reference drug CTX significantly diminished the proteinuria and immune complex deposition in kidney of murine SLE model. MTX failed to inhibit the symptoms.

### Example 1.2 - Confirmation of Efficacy of Laquinimod in the (NZBxNZW)F1 model for SLE - Dose Response Study

This was a survival dose response study to determine whether laquinimod is effective in suppressing the symptoms in (NZBxNZW)F1 mice. The positive control used was Cytoxan.

Seventy-one mice having spontaneous disease developed by the age of 7 months (as measured by proteinuria) were divided into 6 experimental groups (Water, CTX, Laquinimod 0.2 mg/kg, Laquinimod 1.0 mg/kg, Laquinimod 5.0 mg/kg, Laquinimod 25.0 mg/kg) according to their PU scores.

Water and Laquinimod were administered orally (200 µl/mouse) 5 days a week. CTX was administered intraperitonealy once weekly, (200 µl/mouse). Blood samples were collected on weeks 1, 5, 15 and 37. Serum samples were prepared for detection of anti-dsDNA antibodies. After 37 weeks (257 days) of treatment, mice were sacrificed.

The study revealed that laquinimod treatment inhibited the clinical symptoms of disease in NZB/W mice, specifically proteinuria and anti-dsDNA levels resulting in prolonged survival. Treatment with all doses of laquinimod abrogated the progression of proteinuria in comparison to vehicle treatment, while the specific doses of 1, 5, and 25 mg/kg were as effective as the positive control cyclophosphamide (CTX). The dose of 0.2 mg/ml abrogated proteinuria but not to the same extent as compared to the higher doses. In terms of anti-dsDNA levels, there was a dose dependent reduction in antibody levels over time. Finally, all doses resulted in significant prolongation of survival.

### Example 1.3 - Confirmation of Efficacy of Laquinimod in the (NZBxNZW)F1 model for SLE

This study examined the effect of laquinimod (0.2 and 5 mg/kg) versus CTX and vehicle treated (NZBxNZZW)F1 mice.

Seventy mice having spontaneous disease developed by the age of 7 months (as measured by proteinuria) were divided into 4 experimental groups (Water, CTX, Laquinimod 0.2 mg/kg, Laquinimod 5.0 mg/kg,) according to their PU scores.

Water and Laquinimod were administered orally (200 µl/mouse) 5 days a week. CTX was administered intraperitonealy once weekly, (200 µl/mouse). Blood samples were collected on weeks 1, 5, and 11. Serum samples were prepared for detection of anti-dsDNA antibodies. After 13 weeks of treatment, mice were sacrificed and immune complex deposits in their kidneys were evaluated.

This study confirms that laquinimod abrogated disease progression in NZB/W mice as measured by proteinuria. When looking at other endpoints, specifically anti-dsDNA levels and immune complex deposits, treatment with 5 mg/kg behaved similarly to the positive control CTX. Treatment at the low dose (0.2 mg/kg) prevented increased proteinuria but did not inhibit anti-dsDNA Ab titers and ICD.

### Example 1.4 - Non-GLP In Vivo Evaluation of Laquinimod In The MRL/lpr Lupus Mouse Model

This study evaluates the efficacy of laquinimod in the MRL/lpr lupus mouse model.

Animals were monitored until their urine proteinuria reached >200 mg/dL at which time they were enrolled in the study. Animals were dosed with either 1 or 5 mg/kg of laquinimod, p.o., 100 mg/kg mycophenolate mofetil (MMF, CellCept®) p.o., or vehicle (water DDW), p.o., daily except weekends.

Proteinuria, ankle and paw diameters, dsDNA autoantibody levels and survival were monitored during in life portion of the study. At termination, blood samples were harvested for determination of dsDNA autoantibody levels, spleens were harvested and weighed then processed to isolate splenocytes which were counted. Kidney, lung, skin, lymph node, salivary gland and joints were harvested, processed for histological examination and scored by a histopathologist blind to the treatments.

Overall there appeared to be a trend toward dose dependent efficacy in the animals treated with laquinimod in the measures during the in life phase of the experiment. The high variability in the data resulted in these trends not being significant except for some sporadic time points. Histopathological analysis of the kidney reveled significant reductions in kidney glomerulonephritis with MMF and laquinimod treatment at 5 mg/kg treatment compared to vehicle treatment. A significant difference was detected between MMF treatment and vehicle treatment group for the lung BALT hyperplasia. There were no effects of any of the test article treatments on the histopathology of the skin or lymph nodes. When the salivary gland inflammation was evaluated by histopathological scoring, a significant reduction was seen with both MMF and 5 mg/kg laquinimod treatments compared to vehicle treatment. Significant reductions in bone resorption were seen with both doses of laquinimod compared to vehicle control. A significant reduction in cartilage damage was detected with laquinimod treatment at 5 mg/kg compared to vehicle treatment. A significant reduction in inflammation of the joints was seen compared to vehicle control. No significant difference in pannus was detected between any treatment groups. Significant differences were observed between MMF and laquinimod treatment at 5 mg/kg indicating that the higher dose of the test article and MMF treatment were similar. There was a significant reduction in salivary gland inflammation as the higher dose of laquinimod resulted in a significantly lower score than did the lower dose. Joint inflammation was significantly reduced with laquinimod treatment at 5 mg/kg compared to vehicle. There was a trend towards reduction of joint pannus, however there were no other significant differences in the joint parameters between treatment groups. This lack of significant may be due to the high degree of variability in the data. Spleens were weighted and then splenocytes were isolated and counted. The splenocytes were then expressed as a percent of the total spleen cells. The spleen weights showed a trend towards reductions with all test article treatments; however this reduction did not achieve statistical significance. Therefore significant reductions in splenocyte counts with all treatments compared to vehicle. When the splenocytes were expressed as a percent of total spleen cells a significant reduction in percent splenocytes was detected with laquinimod treatment at 5 mg/kg compared to vehicle.

### EXAMPLE 2: Clinical Trial (Phase IIa) - Assessment of Laquinimod For Treatment of Lupus Arthritis

A multicenter, randomized, double-blind, placebo controlled clinical trial is conducted to evaluate the safety, tolerability, biomarkers and clinical effect of laquinimod (0.5 mg/day and 1 mg/day) in Systemic Lupus Erythematosus (SLE) patients with active lupus arthritis.

Although joints, muscles and their supporting structures comprise the most commonly involved system in SLE, very few clinical trials performed to date focus on assessment of this organ system response to treatment. Validated outcome measures often used in clinical trials in SLE (e.g., SLEDAI 2K or BILAG) are not specific or may not be sensitive enough for assessing lupus arthritis activity and its response to treatment. This study assesses lupus arthritis activity by using an organ specific outcome measure - the number of swollen joints.

As suggested by the FDA guidance (FDA, 2005) these musculoskeletal organ specific manifestations is assessed together with general manifestation of SLE, as captured by the BILAG and SLEDAI 2K core.

### Study population and Number of Subjects

Approximately 90 Systemic Lupus Erythematosus patients with active lupus arthritis are enrolled. (approximately 30 subjects per treatment arm). Drop-outs are not replaced.

### Study Duration

The overall study duration is up to 20 weeks, with the screening period being up to 2 weeks, the treatment period being 12 weeks and the follow-up period being 4 weeks.

### Investigational Medicinal Product and Dosage

### Laquinimod/Matching Placebo

Capsules containing laquinimod 0.5 mg and/or matching placebo are administered orally once daily:
1. Laquinimod 0.5 mg arm - 1 capsule of laquinimod 0.5 mg and 1 matching placebo capsule.
2. Laquinimod 1 mg arm - 2 capsules of laquinimod 0.5 mg.
3. Placebo arm - 2 capsules of placebo.

### Inclusion/Exclusion Criteria

### Inclusion Criteria

All subjects must meet all the inclusion criteria below to be eligible:
1. Subjects diagnosed with SLE, who fulfilled at least 4 classification criteria (1997 revised) of the American College of Rheumatology for SLE by the time of screening visit. All subjects should have abnormal titers of anti-nuclear antibodies. [On a case by case basis it is possible to re-assess anti-nuclear antibodies or anti-dsDNA between screening and baseline].
2. Subjects with active lupus arthritis as evident by all of the below:
   a. At least 4 tender and 4 swollen joints at screening and baseline visits [out of the 28 joints assessed].
   b. Moderate or severe arthritis as evident by active synovitis ≥ 1 joints with some loss of functional range of movement, present at screening and baseline visits.
3. Subjects must be between the ages of 18 and 75 years (inclusive).
4. Subject is willing and able to provide a written, informed consent.

### Exclusion Criteria

Any of the following excludes the subject from entering the study:
1. GFR ≤ 30 ml/min/1.73m² as calculated by MDRD formula at screening visit.
2. Subjects with hemoglobin < 8.5 g/dl or neutrophils < 1000/ mm³ or platelets < 50,000/mm³ at screening visit.
3. Any previous diagnosis of drug induced lupus.
4. Subjects with severe, unstable and/or progressive CNS lupus and/or associated with significant cognitive impairment.
5. Subjects with a clinically significant or unstable medical or surgical condition that, in the Investigator's opinion, would preclude safe and complete study participation, as determined by medical history, physical examinations, electrocardiogram (ECG), laboratory tests or imaging. Such conditions may include:
   a. A cardiovascular or pulmonary disorder that cannot be well-controlled by standard treatment permitted by the study protocol.
   b. Metabolic or hematological diseases.
   c. Any form of acute or chronic liver disease including hepatitis B antigen (HBsAg) or anti-hepatitis C virus (anti-HCV) seropositive subjects.
   d. Known Human immunodeficiency virus (HIV) positive status.
   e. Subjects with known active tuberculosis.
   f. Systemic infection at screening.
   g. A history of drug and/or alcohol abuse.
   h. A current major psychiatric disorder.
6. Subjects with a ≥ 2.5x upper limit of normal (ULN) serum elevation of either ALT or AST at screening.
7. Subjects with a ≥ 2x upper limit of normal (ULN) direct or total bilirubin at screening.
8. Medical conditions, other than SLE that requires chronic treatment with immunosuppressive drugs or systemic corticosteroids (not including inhaled steroids).
9. Subjects with a history of malignancy within 5 years from screening with the exception of basal cell carcinoma (completely excised).
10. Women who are pregnant or nursing at the time of screening, or who intend to be during the study period.
11. Women of child-bearing potential who do not practice an acceptable method of birth control [acceptable methods of birth control in this study are: surgical sterilization, intrauterine devices, oral contraceptive, contraceptive patch, long-acting injectable contraceptive, partner's vasectomy, a double-protection method (condom or diaphragm with spermicide)].
12. Subjects treated with oral corticosteroids (e.g prednisolone), who have initiated this treatment within less than 4 weeks prior to screening.
13. Subjects treated with more than 10 mg/day of prednisolone (or equivalent) at baseline, or whose corticosteroid dosage regimen is not stable for at least 2 weeks prior to baseline. [Stable dose defined as ≤ 5 mg prednisolone (or equivalent) increase or decrease, and no IV or IA steroid administration, within the last 2 weeks before baseline.
14. Subjects treated with MTX, who have initiated this treatment within 12 weeks prior to screening.
15. Subjects treated with MTX at doses > 20 mg/week, or who are not on a stable dose for at least 6 weeks prior to screening.
16. Subjects treated with 6-MP, AZA or MMF, who have initiated this treatment within 12 weeks prior to screening or who are not on a stable dose for at least 6 weeks prior to screening.
17. Subjects treated with antimalarial drugs, which are not on a stable dose at screening.
18. New continuous treatment (>3 days or change in dose of continuous treatment with NSAIDs or COX2 inhibitors within 2 weeks prior to baseline.
19. Subjects treated with cyclosporine, IV Ig, abatacept, Leflunomide, plasmaphersis or any biologic agent within 12 weeks prior to screening.
20. Subjects treated with cyclophosphamide or rituximab within 24 weeks prior to screening.
21. Subjects who received any investigational medication within 24 weeks prior to screening.
22. Use of inhibitors of CYP3A4 within 2 weeks prior to baseline visit (1 month for fluoxetine).
23. Use of amiodarone within 2 years prior to screening visit.
24. A known drug hypersensitivity that would preclude administration of study medications, such as known hypersensitivity mannitol, meglumine or sodium stearyl fumarate.
25. Subjects unable to comply with the planned schedule of study visits and study procedures.

### Study Design

This is a Phase IIa, randomized, double-blind, placebo-controlled, study to assess the safety, tolerability, pharmacokinetics, biomarkers and clinical effect of two doses of laquinimod in Systemic Lupus Erythematosus patients with active lupus arthritis. This study evaluates the safety, tolerability of two doses of laquinimod (0.5 mg and 1 mg/day) in Systemic Lupus Erythematosus patients with active lupus arthritis. This study also evaluates biomarkers and clinical effect of laquinimod (0.5 mg and 1 mg/day) in Systemic Lupus Erythematosus patients with active lupus arthritis.

Subjects are assessed for study eligibility up to 2 weeks prior to baseline.

Subjects are initially randomized in a 1:1 ratio into one of the following two treatment arms:
1. Laquinimod 0.5 mg.
2. Matching placebo.

Enrollment to the 1 mg laquinimod dose group is initiated following the approval of the study safety committee, based on data of at least 10 subjects who have completed at least 4 weeks of treatment. Upon approval, randomization into one of the following three treatment arms occurs in a ratio that allows for reaching an overall target enrollment of approximately 30 subjects per treatment arm.
1. Laquinimod 0.5 mg.
2. Laquinimod 1 mg.
3. Matching placebo.

Subjects are allowed to remain on their stable background standard of care medications, according to the study protocol, throughout the trial.

Scheduled in-clinic visits are conducted at screening, baseline and at weeks 2, 4, 8, 12 and 16.

Treatment with laquinimod/placebo is discontinued on visit week 12 and a follow-up/study completion visit is conducted at week 16. Subjects who early discontinued study drug prior to visit week 12 preferably attend a follow-up study completion visit within 4 weeks (28 days) of treatment termination visit.

Unscheduled visits for safety or for any other reason may be conducted at any time during the study.

During the study period the British Isles Lupus Assessment Group (BILAG) score, SLE Disease Activity Index (SLEDAI 2K), Swollen and Tender joint count, Patient Global Assessment score (PGA), Evaluator Global Assessment score (EGA) and Patient Pain assessment (PtP) are assessed in addition to routine safety laboratory and physical tests, PK analysis and disease related immunology tests/biomarkers.

### Allowed Concomitant Medication During Study

The dose of allowed concomitant medications are kept stable throughout the study (from screening to completion of the follow-up period, as defined in the study protocol). Any new medication/treatment for SLE or dose increase not allowed by the study protocol, throughout the study treatment period, results in major protocol violation and is regarded as a treatment failure. Decrease in dose or dose regimen, not allowed by the study protocol, throughout the study treatment period, also result in major protocol violation. Further, any new biologic treatment, new immunosuppressive drug or cytotoxic drug, plasmapheresis or IV-Ig administered to subjects at any time throughout the study treatment period, is regarded as treatment failure and result in early treatment discontinuation.

### Corticosteroids

The allowed background dose of oral corticosteroids (up to 10 mg prednisone/prednisolone or equivalent) remains stable throughout the study. Stable dose is defined as < 5 mg prednisone/prednisolone (or equivalent) change compared to baseline. IV, IM or Intraarticular (IA) dose are not allowed.

### Immunosuppressives

1. Immunosuppressive treatment allowed by the study protocol (AZA, 6MP, MTX, MMF) is kept stable throughout the study. Treatment with a new immunosuppressive or cytotoxic drug during the treatment period results in early treatment discontinuation and is regarded as a treatment failure. Dose increase during the treatment period is regarded as treatment failure.
2. Treatment with any new biologic treatment (e.g., abatacept, anti-TNFs, Rituximab, other), throughout the treatment period, is regarded as major protocol violation and treatment failure and results in early treatment discontinuation.

### Other

1. Treatment with anti-inflammatory drugs (NSAIDs) or COX2 inhibitors are kept stable during the trial. New treatment or change in dose throughout the treatment period is regarded as a treatment failure.
2. Treatment with non steroidal anti inflammatory drugs (NSAIDs) or COX2 inhibitors are kept stable during the trial. New continuous treatment (>3 days) with NSAIDs or COX2 inhibitors, throughout the treatment period, is regarded as protocol violation and a treatment failure. Treatment per-need (≤ 3 days continuous treatment) is allowed.
3. Bone protection therapy (e.g. bisphosphonates) is allowed throughout the trial.
4. The use of CYP1A2 substrates (e.g. Warfarin) during the treatment period is permitted, however subjects treated with these medications should be monitored for possible reduction in their effect.

### Follow-up period

All attempts are made to maintain a stable dose of background medications (e.g. NSAIDs, COX2 inhibitors, antimalarials, steroids, immunosuppressives) or any other drug prescribed during the treatment period, throughout the follow up period.

### Disallowed Concomitant Medications during Study

1. No drugs for the treatment of lupus arthritis other than those listed above are allowed during the course of the study.
2. Rescue therapy for SLE (any new medication/ treatment or dose increase, not allowed by the protocol), throughout the study treatment period, results in major protocol violation and is regarded as a treatment failure. Any new biologic treatment or new immunosuppressive or cytotoxic drug, IV-Ig or plasmapheresis, throughout the study treatment period, is regarded as treatment failure and results in early treatment discontinuation.
3. Decrease in dose or dose regimen, not allowed by the protocol, throughout the study treatment period, results in major protocol violation.
4. New continuous treatment (>3 days) with NSAIDs or COX2 inhibitors is regarded as protocol violation and a treatment failure.
5. Inhibitors of CYP3A4 are not allowed throughout the study (2 weeks prior to baseline to the end of the follow up period). In case of treatment discontinuation of laquinimod, special attention should be paid to avoid drugs that are CYP3A4 inhibitors for up to 30 days.

### PK Analysis

### Pharmacokinetic (PK)lPopulation PK Study (PPK):

Blood samples for PK evaluation is collected from all subjects as follows: Visit Week 4 - full PK profile at the following times: pre-dose, 15, 30 min and 1, 1.5, 2, 3, 4, 6, and 24 hours post dosing; Visit weeks 2 and 12 - prior to dosing (trough plasma levels).

### Monitoring Plan and Safety Stopping Rules

In any of the events listed below, the subject's participation in the study is discontinued immediately. The subject is followed until resolution or stabilization of symptoms or lab abnormalities:

### Liver function tests:

1. Any increase in ALT or AST to ≥3 times ULN, combined with either of the following:
   a. ≥1.5 times ULN elevation of INR for subjects not treated with Warfarin.
   b. Significant elevation of INR (per Investigator discretion, compared with usual target INR) for subjects treated with Warfarin.
   c. Elevation ≥2 times ULN of total bilirubin (and the lack of evidence of haemolysis (raised reticulocyte count or reduced haptoglobulins).
   d. Any increase in ALT or AST to ≥3 times ULN, with the appearance of worsening of nausea, vomiting, fever, rash, or eosinophilia.
   e. Any increase in ALT or AST to levels ≥5 but <8 times ULN, which is persistent for ≥2 weeks of repeated measurements.
   f. Any increase in ALT or AST to levels ≥8 times ULN. *Hematologic Abnormalities (SLE related)*

1. Neutropenia - absolute neutrophil count < 1000/mm³.
2. Thrombocytopenia - PLT < 50,000/mm³ for 2 consecutive visits (at least 2 weeks apart).
3. Hgb < 8 gr/DL - for 2 consecutive visits (at least 2 weeks apart).

### Withdrawal Criteria/Treatment Failure

1. Subjects with 50% increase in swollen or tender joint counts compared to baseline at any time during the study are regarded as a treatment failure and withdrawn from the study.

### Outcome Measures

### Response Definitions

▪ BILAG MSK response is defined as change from Musculoskeletal A or B at baseline to C or D at LOV.
▪ BILAG Substantial Responder (SR) is defined as all systems at last observed value (LOV) are C or D/E providing at least one system is A or B at baseline.
▪ SLEDAI 2K response - decrease in SLEDAI of at least 4 point compared to baseline
▪ Medicinal/ Interventional flare defined as any of the following
   □ Steroid increase by at least 5mg/day compared to previous dose or compared to baseline or any IV, IM or Intraarticular dose.
   □ New continuous treatment (>3days) or increase in dose of NSAIDs or COX2 inhibitors.
   □ New treatment or dose increase with an immunosuppressive drug compared to previous dose or compared to baseline.
   □ Treatment with biologic agents, IVIG or plasmapheresis.
   □ New treatment or dose increase of antimalarial drugs compared to previous dose or compared to baseline.

### Clinical Effect Outcome Measure

### Lupus Arthritis

### 1. Change in swollen joint counts at week 12

Descriptive statistics of swollen joint counts at week 12 as well as change from baseline are presented by treatment group in tabular and graphical forms.

### 2. Change in tender joint counts at week 12

Descriptive statistics of tender joint counts at week 12 as well as change from baseline are presented by treatment group in tabular and graphical forms.

### 3. Change in swollen and tender joint counts at week 12

Descriptive statistics of tender joint counts plus swollen joint counts at week 12 as well as change from baseline are presented by treatment group in tabular and graphical forms.

### 4. Proportion of BILAG Musculoskeletal (MSK) response at week 12 and the lack of treatment failure

The number and percent of subjects, calculated from the randomized population, who are in BILAG MSK response at Week 12 and did not experience treatment failure, are presented both in tabular and graphical forms by treatment group.

Lupus arthritis is characterized by joint tenderness and swelling. The number of tender and swollen joints are be used to assess lupus arthritis activity. "Joint tenderness" is defined as the presence of tenderness and/or pain in a joint at rest with pressure or on passive movement of the joint/joint manipulation. "Joint swelling" is soft tissue swelling that is detectable along the joint margins.

### General SLE

### 1. Proportion of substantial BILAG responders at Week 12 and the lack of treatment failure

The number and percent of subjects, calculated from the randomized population, who are with substantial BILAG response at Week 12 and did not experience treatment failure, are presented both in tabular and graphical forms by treatment group.

### 2. Proportions of SLEDAI 2K responders at week 12 and the lack of treatment failure

The number and percent of subjects, calculated from the randomized population, who are with SLEDAI response at week 12 and did not experience treatment failure, are presented both in tabular and graphical forms by treatment group.

### 3. Proportion of subjects with new BILAG A or B anytime during the treatment period

The number and percent of subjects, calculated from the randomized population, who experienced a new BILAG A or B in any system throughout the treatment period (12 weeks), are presented both in tabular and graphical forms by treatment group.

### 4. Proportion of subjects with new medicinal/interventional flare throughout the treatment period

The number and percent of subjects, calculated from the randomized population, who are with new medicinal interventional flare anytime during the treatment period, are presented both in tabular and graphical forms by treatment group.

### 5. Change from baseline to week 12 in Patient and Evaluator Global Assessment (PGA & EGA)

Descriptive statistics of PGA and EGA at Week 12 as well as change from baseline are presented by treatment group in tabular and graphical forms.

### 6. Change from baseline in SLEDAI 2K

Descriptive statistics of SLEDAI 2K as well as change from baseline are presented by week in trial and treatment group in tabular and graphical forms.

### 7. Change in anti-dsDNA , C3, C4 and CH50

Descriptive statistics of anti-dsDNA, C3, C4, CH50 as well as change from baseline will be presented by presented by week in trial and treatment group in tabular and graphical forms. Similarly, the number and percent of subjects shifted from normal at baseline to abnormal are presented by week in trial and treatment group in tabular forms.

### 8. Cytokines and Chemokines (Serum, PBMC's supernatant), gene expression and cell surface markers (PBMC's) at week 12

Descriptive statistics of biomarkers at Week 12 as well as change from baseline are presented by week in trial and treatment group in tabular and graphical forms.

Both joint tenderness and swelling are dichotomic measures (swollen versus non swollen and tender versus non tender).

Sixty-eight (68) joints are examined for swelling at all study visits (including Screening, Baseline and visit weeks 2, 4, 8, 12 and 16). These joints include: Temporomandibular (n=2), Sternoclavicular (n=2), Acromioclavicular (n=2), Shoulder (R&L, n=2), Elbow (R&L, n=2), Wrist (R&L, n=2), Metacarpophalageal (R&L X5, n=10), Interphalangeal of thumb (n=2), distal interphalangeal (n=2), Proximal interphalangeal (n=8), hip (n=2), Knee (R&L, n=2), ankle mortise (n=2), ankle tarsus (n=2), metatarsophalangeal (n=10), interphalangeal of great toe (n=2), and proximal/distal interphalangeal of the toes (n=8).

Sixty-six (66) joints are examined for swelling at all study visits. These joints include: Temporomandibular (n=2), Sternoclavicular (n=2), Acromioclavicular (n=2), Shoulder (R&L, n=2), Elbow (R&L, n=2), Wrist (R&L, n=2), Metacarpophalageal (R&L X5, n=10), Interphalangeal of thumb (n=2), distal interphalangeal (n=2), Proximal Interphalangeal (n=8), Knees (R&L, n=2), ankle mortise (n=2), ankle tarsus (n=2), metatarsophalangeal (n=10), interphalangeal of great toe (n=2), and proximal/distal interphalangeal of the toes (n=8).

Subjects with at least 4 tender and 4 swollen joints at screening and baseline visits (out of the 28 joints assessed), are be eligible for this study.

Patient Global Assessment (PGA) is a Visual Analogue Scale. It measures the subject perception of his/hers overall health condition, from very well to very poor. PGA is performed at every visit (except for screening). It is important that the patient global assessment is collected as early as possible at any visit, before other planned visit activities/evaluations are being done in order to minimize potential influence on the patient perspective.

Evaluator/physician Global Assessment (EGA) is a Visual Analogue Scale. It measures the disease activity based on the physician subjective assessment from none active to worse disease activity. EGA is performed at every visit (except for screening).

### Safety and Tolerability Outcome Measures

### Safety

1. Incidence, frequency and severity of adverse events (AEs).
2. Change in clinical laboratory values.
3. Change in vital signs.
4. Change in ECG.

The incidence and frequency of adverse events are presented by System Organ Class, High Level Grouped Term, High Level Term and preferred terminology according to MedDRA dictionary.

### Tolerability

1. Proportion of subjects who prematurely discontinue treatment.
2. Proportion of subjects who prematurely discontinue treatment due to AEs.
3. Time to premature treatment discontinuation.
4. Time to premature treatment discontinuation due to AEs.

Tolerability analysis is based on the number (%) of subjects who failed to complete the study, the number (%) of subjects who failed to complete the study due to adverse events. Time to withdrawal is presented by Kaplan-Meier curves.

### Results

This study assesses the efficacy, tolerability and safety of daily dose of 0.5 mg and 1.0 mg laquinimod as compared to placebo in Systemic Lupus Erythematosus (SLE) patients with active lupus arthritis.

Daily oral administration of 0.5 mg or 1 mg laquinimod reduces the subject's swollen joint count during the study period as compared to the administration of placebo.

Daily oral administration of 0.5 mg or 1 mg laquinimod reduces the subject's tender joint count during the study period as compared to the administration of placebo.

Daily oral administration of 0.5 mg or 1 mg laquinimod improves the subject's BILAG MSK response during the study period as compared to the administration of placebo.

Daily oral administration of 0.5 mg or 1 mg laquinimod improves the subject's BILAG score during the study period as compared to the administration of placebo.

Daily oral administration of 0.5 mg or 1 mg laquinimod lowers the C-Reactive Protein level, serum cytokine level, serum chemokine level and/or anti-dsDNA level of the subject during the study period.

Daily oral administration of 0.5 mg or 1 mg laquinimod reduces the subject's Disease Activity Score using 28 joint counts (DAS28), 66 swollen/68 tender joint count (JC66/68) and/or reduces the subject's physician global assessment (PGA) score during the study period.

Daily oral administration of 0.5 mg or 1 mg laquinimod is well tolerated and has no toxicity as compared to the administration of placebo.

### References

1. "Systemic Lupus Erythematosus" The Merck Manual, 17th ed. Mark H. Beers, MD, Robert Berkow, MD, eds. Whitehouse Station, NJ: Merck Research Labs, 1999.
2. 0130282 99506202. A double blind, randomised, repeat-dose, dose escalation study of ABR-215062 versus placebo in healthy volunteers and patients with multiple sclerosis. Active Biotech Research AB, Sweden. Final Clinical Trial Report, January 2002.
3. 03506207. An open safety study on laquinimod (ABR-215062) in patients with multiple sclerosis. Active Biotech Research AB, Sweden. Final Clinical Trial Report, April 2007.
4. 0430067 275-1061-01. Determination of the effects of ABR-212616, ABR-215050, ABR-215062 and ABR-215757 on the activities of CYP1A2 and CYP3A4 in cryopreserved human hepatocytes. In Vitro Technologies, USA. Final Report, February 2004.
5. 0430518 275-1081-02. Determination of the effects of ABR-215062 on CYP1A2 and CYP3A4 in cryopreserved human hepatocytes. In Vitro Technologies, USA. Final Report, August 2004.
6. 9830089. PNU-215045, PNU-215062: Effects on cytochrome P450 enzymes in female Sprague Dawley rats. Lund Research Center AB, Active Biotech Group, Sweden. Final Report, November 1998.
7. 9830133. PNU-215062: Effects on cytochrome P450 enzymes in female Sprague Dawley rats. Lund Research Center AB, Active Biotech Group, Sweden. Final Report, November 1998.
8. A two-period, open-label, one-sequence crossover study in healthy subjects to assess the potential interaction of fluconazole on laquinimod pharmacokinetics. PRACS Institute Cetero Research, ND, USA. Final Report, June 2009.
9. Appel GB Dooley MA Ginzler EM. Mycophenolate mofetil compared with intravenous cyclophosphamide as induction therapy for lupus nephritis: Aspreva Lupus Management Study (ALMS) results. 47A of JASN, Vol 18 October 2007.
10. Austin HA, Balow JE. Diffuse proliferative Lupus Nephritis: Identification of specific pathologic features affecting renal outcomes. Kidney International 1984; 25:689-695.
11. Bevra Hahn. Systemic Lupus Erythematosus. In: Braunwald E., Fauci AS, Kasper DL, Hauser SL, Longo DL, Jameson JL, eds. Harrison's Principles of Internal Medicine. New York: McGraw-Hill Professional, 2001:1922-28.
12. Boumpas DT. Optimum therapeutic approaches for Lupus Nephritis: What Therapy and foe whom. Nature Clinical Practice Rheumatology. 2005;1: 22-30.
13. Brent LH. Lupus Nephritis, Emedicine, 2008.
14. Chan TM Li FK Tang CS. Efficacy of mycofenolate mofetil in patients with diffuse proliferative Lupus Nephritis. N Eng J Med; 2000;343: 1156-1162.
15. FDA 2005. Draft Guidance for Industry - Systemic Lupus Erythematosus - Developing Drugs for Treatment (http://www.fda.gov/downloads/Drugs/GuidanceComplianceRegulatory Information/Guidances/ucm072063.pdf).
16. Ginzler EM Dooley MA Aranow C. Mycophenolate Mofetil or intravenous Cyclophosphamide for Lupus Nephritis. N Eng J Med; 2005;353: 2219-2228.
17. Grossman, J.M. et al. (2009) "Lupus Arthritis" Best Practice & Research Clinical Rheumatology. August 2009, 23(4):495-506.
18. Isenberg DA Appel GB Posley MA . Mycophenolate mofetil compared with intravenous cyclophosphomite an induction for lupus nephritis: ALMS results and BILAG responses. Ann Rheum Dis 2008; 67: S: 53-54.
19. Kurucz I., S. Toth, K. Nemeth, K. Torok, V. Csillik-Perczel, A. Pataki, C. Salamon, Z. Nagy, J.I. Szekely, K. Horvath, and N. Bodor (2003) "Potency and specificity of the pharmacological action of a new, antiasthmatic, topically administered soft steroid, etiprednol dicloacetate (BNP-166)". J Pharmacol Exp Ther. 307(1):83-92.
20. Merill J at al. Mycophenolate Mofetil (MMF) Is Effective for Systemic Lupus (SLE) Arthritis, Final Results of An Organ-Specific, Double-Blind, Placebo-Controlled Trial. Arthritis Rheum 2009;60 Suppl 10:264.
21. Petri M. Epidemiology of systemic lupus erythematosus. Best Pract Res Clin Rheumatol 2002;16(5):847-858.
22. Polman, C. et al., (2005) "Treatment with laquinimod reduces development of active MRI lesions in relapsing MS", Neurology. 64:987-991.
23. Sandberg-Wollheim M, et al. (2005) "48-week open safety study with high-dose oral laquinimod in patients", Mult Scler. 11:S154 (Abstract).
24. Sharabi A. A. Haviv, H. Zinger, M. Dayan and E. Moses (2006) "Amelioration of murine lupus by a peptid, based on the complementarity determining region 1 of an autoantibody as compared to dexamethasone: different effects on cytokines and apoptosis". Clin. Immunology. 119:146-155.
25. Sharabi A., H. Zinger, M. Zborowsky, Z.m. Sthoeger and E. Mozes (2006) "A peptide based on the complementarity-determining region 1 of an autoantibody ameliorates lupus by up-regulating CD4+CD25+ cells and TGB-B". PNAS 1103:8810-8815.
26. The American College of Rheumatology response criteria for proliferative and membranous renal disease in Systemic Lupus Erythemtosus. Arthritis Rheum; 54(2): 421-432.
27. TQT-LAQ-122. A Double-Blind, Randomized, Parallel Group, Thorough QT/QTc Trial in Healthy Men and Women to Assess the Effect of Laquinimod on Cardiac Repolarization Using a Clinical and a Supratherapeutic Dose Compared to Placebo, with Moxifloxacin as a Positive Control. PRACS Institute Cetero Research, ND, USA. Final Report, June 2009.
28. U.S. Patent No. 6, 077, 851, issued June 20, 2000 to Bjork, et al.
29. Wallace D.J. in Dubois' Lupus Eryhthematosus, the Musculoskeletal System. Lippincott Williams& Wilkins, 7th edition; 2007: 647-661.
30. Weening JJ et al on behalf of the International Society of Nephrology and Renal Pathology Society Working Group on the classification of lupus nephritis. The classification of glomerulonephritis in systemic lupus erythematosus revisited. Kidney International Journal 2004, 67; 521-530.
31. Yee CS, Caroline Gordon, et al. British Isles Lupus Assessment Group 2004 Index is valid for assessment of disease activity in SLE. Arthritis &Rheumatism. 2007; 56:4113-4119.
32. Yee CS, Caroline Gordon, et al. British Isles Lupus Assessment Group 2004 Index. A reliable tool for assessment of SLE activity. Arthritis &Rheumatism. 2006; 54:3300-3305.

## Claims

1. Laquinimod or a pharmaceutically acceptable salt thereof for use in treating a subject afflicted with active lupus arthritis.

2. A pharmaceutical composition comprising an amount of laquinimod or a pharmaceutically acceptable salt thereof for use in treating a subject afflicted with active lupus arthritis.

3. Laquinimod or a pharmaceutically acceptable salt thereof for use according to claim 1, wherein the treatment comprises periodically administering to the subject an amount of laquinimod or of the pharmaceutically acceptable salt effective to treat the subject.

4. Laquinimod or a pharmaceutically acceptable salt thereof for use according to claim 1 wherein the amount of laquinimod or of the pharmaceutically acceptable salt is effective to reduce a clinical sign or symptom of active lupus arthritis in the subject.

5. A pharmaceutically acceptable salt of laquinimod for use according to claim 3 or 4, wherein the pharmaceutically acceptable salt is laquinimod sodium.

6. Laquinimod or a pharmaceutically acceptable salt thereof for use according to claim 3 or 5, wherein the periodic administration of laquinimod or of the pharmaceutically acceptable salt is effected orally.

7. Laquinimod or a pharmaceuticallly acceptable salt thereof for use according to claim 6, wherein the amount of laquinimod administered is 0.5-1.0 mg/day, preferably 0.5 mg/day or 1.0 mg/day.

8. Laquinimod or a pharmaceutically acceptable salt thereof for use according to claim 3 or 4, wherein the treatment further comprises administering a corticosteroid, an immunosuppressive, an anti-malarial drug, a non-steroid anti-inflammatory drug, a COX2 inhibitor, abatacept, rituximab, and/or belimumab.

9. Laquinimod or a pharmaceutically acceptable salt thereof for use according to claim 8, wherein the immunosuppressive drug is azathioprine, methotrexate, 6-mercaptopurine, leflunomide, cyclosporine or other calcineurin inhibitors.

10. Laquinimod or a pharmaceutically acceptable salt thereof for use according to claim 3 or 4, wherein the periodic administration continues for at least 12 weeks.

11. Laquinimod or a pharmaceutically acceptable salt thereof for use according to or any one of claims 3-7 or 10, wherein laquinimod or the pharmaceutically acceptable salt thereof is administered as monotherapy for active lupus arthritis.

12. Laquinimod or a pharmaceutically acceptable salt thereof for use according to any one of claims 3-10, wherein laquinimod or the pharmaceutically acceptable salt thereof is administered as adjunct therapy with another active lupus arthritis treatment.

13. Laquinimod or a pharmaceutically acceptable salt thereof for use according to any one of claims 3-12, wherein the periodic administration of laquinimod or of the pharmaceutically acceptable salt reduces the subject's swollen joint count.

14. Laquinimod or a pharmaceutically acceptable salt thereof for use according to any one of claims 3-13, wherein the periodic administration of laquinimod or of the pharmaceutically acceptable salt reduces the subject's tender joint count.

15. Laquinimod or a pharmaceutically acceptable salt thereof for use according to any one of claims 3-14, wherein the periodic administration of laquinimod or of the pharmaceutically acceptable salt improves the subject's BILAG MSK response.

## Patentansprüche

1. Laquinimod oder pharmazeutisch annehmbares Salz davon zur Verwendung bei der Behandlung eines Subjektes, das an einem aktiven Lupus Arthritis leidet.

2. Pharmazeutische Zusammensetzung umfassend eine Menge an Laquinimod oder einem pharmazeutisch annehmbaren Salz davon zur Verwendung bei der Behandlung eines Subjektes, das ein einem aktiven Lupus Arthritis leidet.

3. Laquinimod oder ein pharmazeutisch annehmbares Salz davon zur Verwendung nach Anspruch 1, wobei die Behandlung die periodische Verabreichung einer Menge von Laquinimod oder eines pharmazeutisch annehmbaren Salzes, die zur Behandlung des Subjektes wirksam ist, an ein Subjekt umfasst.

4. Laquinimod oder ein pharmazeutisch annehmbares Salz davon zur Verwendung nach Anspruch 1, wobei die Menge an Laquinimod oder des pharmazeutisch annehmbaren Salzes davon wirksam ist, ein klinisches Signal oder Symptom eines aktiven Lupus Arthritis bei dem Subjekt zu verringern.

5. Pharmazeutisch annehmbares Salz von Laquinimod zur Verwendung nach Anspruch 3 oder 4, wobei das pharmazeutisch annehmbare Salz Laquinimodnatrium ist.

6. Laquinimod oder ein pharmazeutisch annehmbares Salz davon zur Verwendung nach Anspruch 3 oder 5, wobei die periodische Verabreichung von Laquinimod oder dem pharmazeutisch annehmbaren Salz oral durchgeführt wird.

7. Laquinimod oder ein pharmazeutisch annehmbares Salz davon zur Verwendung nach Anspruch 6, wobei die Menge an verabreichtem Laquinimod 0,5 bis 1,0 mg/Tag, bevorzugt 0,5 mg/Tag oder 1,0 mg/Tag beträgt.

8. Laquinimod oder ein pharmazeutisch annehmbares Salz davon zur Verwendung nach Anspruch 3 oder 4, wobei die Behandlung ferner die Verabreichung eines Corticosteroids, eines Immunsuppressivums, eines Antimalariamittels, eines nicht-steroiden entzündungshemmenden Mittels, eines COX2-Inhibitors, Abatacept, Rituximab, und/oder Belimumab umfasst.

9. Laquinimod oder ein pharmazeutisch annehmbares Salz davon zur Verwendung nach Anspruch 8, wobei das Immunsuppressivum Azathioprin, Methotrexat, 6-Mercaptopurin, Leflunomid, Cyclosporin oder anderer Calcineurininhibitoren sind.

10. Laquinimod oder ein pharmazeutisch annehmbares Salz davon zur Verwendung nach Anspruch 3 oder 4, wobei die periodische Verabreichung mindestens 12 Wochen lang andauert.

11. Laquinimod oder ein pharmazeutisch annehmbares Salz davon zur Verwendung nach einem der Ansprüche 3 bis 7 oder 10, wobei Laquinimod oder das pharmazeutisch annehmbare Salz davon als Monotherapie für aktiven Lupus Arthritis verabreicht wird.

12. Laquinimod oder ein pharmazeutisch annehmbares Salz davon zur Verwendung nach einem der Ansprüche 3 bis 10, wobei Laquinimod oder das pharmazeutisch annehmbare Salz davon als Zusatztherapie zu einer anderen aktiven Behandlung eines Lupus Arthritis verabreicht wird.

13. Laquinimod oder ein pharmazeutisch annehmbares Salz davon zur Verwendung nach einem der Ansprüche 3 bis 12, wobei die periodische Verabreichung von Laquinimod oder des pharmazeutisch annehmbaren Salzes die Anzahl geschwollener Gelenke des Subjekts verringert.

14. Laquinimod oder ein pharmazeutisch annehmbares Salz davon zur Verwendung nach einem der Ansprüche 3 bis 13, wobei die periodische Verabreichung von Laquinimod oder des pharmazeutisch annehmbaren Salzes die Anzahl druckschmerzempfindlicher Gelenke des Subjekts verringert.

15. Laquinimod oder ein pharmazeutisch annehmbares Salz davon zur Verwendung nach einem der Ansprüche 3 bis 14, wobei die periodische Verabreichung von Laquinimod oder dem pharmazeutisch annehmbaren Salz die BILAG-MSK-Antwort des Subjekts verbessert.

## Revendications

1. Laquinimod ou un sel pharmaceutiquement acceptable de ce dernier pour une utilisation dans le traitement d'un sujet souffrant d'arthrite du lupus active.

2. Composition pharmaceutique comprenant une quantité de laquinimod ou d'un sel pharmaceutiquement acceptable de ce dernier pour une utilisation dans le traitement d'un sujet souffrant d'arthrite du lupus active.

3. Laquinimod ou un sel pharmaceutiquement acceptable de ce dernier pour une utilisation conforme à la revendication 1, où le traitement comprend l'administration périodique au sujet d'une quantité de laquinimod ou du sel pharmaceutiquement acceptable de ce dernier efficace pour traiter le sujet.

4. Laquinimod ou un sel pharmaceutiquement acceptable de ce dernier pour une utilisation conforme à la revendication 1, où la quantité de laquinimod ou du sel pharmaceutiquement acceptable de ce dernier est efficace pour réduire un signe ou un symptôme clinique de l'arthrite du lupus active chez le sujet.

5. Sel pharmaceutiquement acceptable de laquinimod pour une utilisation conforme à la revendication 3 ou 4, lequel sel pharmaceutiquement acceptable est du sodium de laquinimod.

6. Laquinimod ou un sel pharmaceutiquement acceptable de ce dernier pour une utilisation conforme à la revendication 3 ou 5, où l'administration périodique de laquinimod ou du sel pharmaceutiquement acceptable de ce dernier est effectuée oralement.

7. Laquinimod ou un sel pharmaceutiquement acceptable de ce dernier pour une utilisation conforme à la revendication 6, où la quantité de laquinimod administrée est de 0,5 à 1,0 mg / jour, de préférence de 0,5 mg / jour ou de 1,0 mg / jour.

8. Laquinimod ou un sel pharmaceutiquement acceptable de ce dernier pour une utilisation conforme à la revendication 3 ou 4, où le traitement comprend en outre l'administration d'un corticostéroïde, d'un immunosuppresseur, d'un médicament anti-malaria, d'un médicament anti-inflammatoire non stéroïdien, d'un inhibiteur de la COX-2, d'abatacept, de rituximab, et / ou de belimumab.

9. Laquinimod ou un sel pharmaceutiquement acceptable de ce dernier pour une utilisation conforme à la revendication 8, où le médicament immunosuppresseur est de l'azathioprine, du méthotrexate, de la 6-mercaptopurine, du léflunomide, de la cyclosporine ou d'autres inhibiteurs de la calcineurine.

10. Laquinimod ou un sel pharmaceutiquement acceptable de ce dernier pour une utilisation conforme à la revendication 3 ou 4, où l'administration périodique se poursuit durant au moins 12 semaines.

11. Laquinimod ou un sel pharmaceutiquement acceptable de ce dernier pour une utilisation conforme à l'une quelconque des revendications 3 à 7 ou 10, lequel laquinimod ou sel pharmaceutiquement acceptable de ce dernier est administré en tant que monothérapie pour l'arthrite du lupus active.

12. Laquinimod ou un sel pharmaceutiquement acceptable de ce dernier pour une utilisation conforme à l'une quelconque des revendications 3 à 10, lequel laquinimod ou sel pharmaceutiquement acceptable de ce dernier est administré en tant que thérapie d'appoint à un autre traitement de l'arthrite du lupus active.

13. Laquinimod ou un sel pharmaceutiquement acceptable de ce dernier pour une utilisation conforme à l'une quelconque des revendications 3 à 12, où l'administration périodique du laquinimod ou du sel pharmaceutiquement acceptable réduit le nombre d'articulations enflées du sujet.

14. Laquinimod ou un sel pharmaceutiquement acceptable de ce dernier pour une utilisation conforme à l'une quelconque des revendications 3 à 13, où l'administration périodique du laquinimod ou du sel pharmaceutiquement acceptable réduit le nombre d'articulations douloureuses du sujet.

15. Laquinimod ou un sel pharmaceutiquement acceptable de ce dernier pour une utilisation conforme à l'une quelconque des revendications 3 à 14, où l'administration périodique du laquinimod ou du sel pharmaceutiquement acceptable améliore la réponse du sujet selon le BILAG MSK.
